# EUROPÄISCHE PATENTANMELDUNG

(11) **EP 1 518 533 A2**
(43) Veröffentlichungstag der Anmeldung: **30.03.2005**
(21) Anmeldenummer: 04012315.0
(22) Anmeldetag: 25.05.2004
(51) Int. Cl.: A61K 7/00, A61K 9/12

(54) **Sprühbare kosmetische Formulierung**

(30) Priorität: 23.09.2003 DE 10343851
(71) Anmelder: Beiersdorf AG, 20253 Hamburg (DE)
(72) Erfinder: Kröpke, Rainer, 22869 Schenefeld (DE); Riedel, Heidi, 22529 Hamburg (DE); Syskowski, Boris, 22303 Hamburg (DE)

(57) **Zusammenfassung**

Die vorliegende Erfindung betrifft eine gelförmige, sprühbare kosmetische Formulierung.

## Beschreibung

Die vorliegende Erfindung betrifft gelförmige, feindisperse Systeme vom Typ ÖI-in-Wasser und Hydrodispersionsgele bevorzugt als kosmetische oder dermatologische Zubereitungen.

Gele sind formbeständige, leicht deformierbare, an Flüssigkeiten u. Gasen reiche disperse Systeme aus mindestens zwei Komponenten, die zumeist aus einem festen, kolloidal zerteilten Stoff mit langen oder stark verzweigten Teilchen (z. B. Gelatine, Kieselsäure, Montmorillonit, Bentonite, Polysaccharide, Pektine u. a., oft als Verdickungsmittel bezeichnete *Geliermittel*) und einer Flüssigkeit (meist Wasser) als Dispersionsmittel bestehen. Dabei ist die feste Substanz kohärent, d. h. sie bildet im Dispersionsmittel ein räumliches Netzwerk, wobei die Teilchen durch Neben- od. Hauptvalenzen an verschiedenen Punkten aneinanderhaften. Sind die Zwischenräume zwischen den Teilchen mit Flüssigkeit ausgefüllt, so liegt ein *Lyogel* (*Gallerte*) vor, dass sich durch Energiezufuhr verflüssigen lässt, z. B. durch Rühren (Erscheinung der Thixotropie). Besteht das Dispersionsmittel aus Wasser, so spricht man von *Hydrogelen.* Formbeständige Hydrogele entstehen z. B. schon aus 0,2% Agar und 99,8% Wasser oder 0,6% Gelatine u. 99,4% Wasser.
Auch aus Solen können sich Gele bilden, u. zwar durch einen Flockung od. Ausflockung genannten Vorgang. *Xerogele* sind Gele, die ihre Flüssigkeit auf irgendeine Weise (durch Verdampfen, Abpressen oder Absaugen) verloren haben, wobei sich auch die räumliche Anordnung des Netzes verändert, so dass die Abstände zwischen den Strukturelementen nur noch Dimensionen von Atomabständen besitzen (*Beispiel:* eingetrocknete Gelatine, Leim, Agar-Agar). Bei Xerogelen handelt es sich um einen Grenzzustand zum Festkörper. Xerogele im chemischen Laboratorium sind die Kieselgele. Durch Quellung (bei Zugabe des Dispersionsmittels) können die Xero- wieder in Lyogele übergehen.
Gele können *inkohärente Systeme* (jedes Teilchen ist als "kinetische Einheit" frei beweglich und hängt mit keinem anderen Teilchen zusammen) oder *kohärente Systeme* (die dispergierte Substanz hängt hier infolge der Kohäsion irgendwie, z. B. netzartig, zusammen und ist nicht mehr frei beweglich). Diese Definitionen sind identisch mit der Einteilung in *Sole* (inkohärent) u. *Gele* (kohärent). Bei Solen pflegt man die Art der Dispersionsmittel zur Kennzeichnung zuzufügen, z. B. Hydrosole u. Organosole als *Lyosole,* Aerosole. Sole haben Flüssigkeits- (bzw. Gas-) Eigenschaften. Bei Gelen unterscheidet man flüssigkeitshaltige *Lyogele* (wobei es wieder Hydrogele, Sulfogele usw. gibt) und *Xerogele,* die (wie z. B. Kieselsäure-Gel oder Gelatine-Blättchen) keine Flüssigkeit enthalten. Mit Gasen als Dispersionsmittel erhält man *Aerogele.*
Die *physikal.-chem. Eigenschaften* von Dispersionen unterscheiden sich beträchtlich von denen anderer Substanzen. Sie sind insbesondere abhängig von Größe, Gestalt u. Struktur der Partikel. Emulsionen z. B. erscheinen klar-durchsichtig, wenn sie nur Teilchen mit <0,01 µm, dagegen milchig-trüb, wenn sie Teilchen mit >0,1 µm Durchmesser enthalten.

Unter Emulsionen versteht man im Allgemeinen heterogene Systeme, die aus zwei nicht oder nur begrenzt miteinander mischbaren Flüssigkeiten bestehen, die üblicherweise als Phasen bezeichnet werden. In einer Emulsion ist eine der beiden Flüssigkeiten in Form feinster Tröpfchen in der anderen Flüssigkeit dispergiert.

Sind die beiden Flüssigkeiten Wasser und ÖI und liegen Öltröpfchen fein verteilt in Wasser vor, so handelt es sich um eine ÖI-in-Wasser-Emulsion (O/W-Emulsion, z. B. Milch). Der Grundcharakter einer O/W-Emulsion ist durch das Wasser geprägt.
Eine sehr elegante Form er ÖI-in-Wasser-Emulsion ist die Silikon-in-Wasser-Emulsion. Hier werden keine traditionellen Lipide emulgiert, sondern überwiegend Silikonöle (zyklische und/oder lineare Silikonöle), die ein seidiges, leichtes Hautgefühl ergeben. Bei einer Wasser-in-ÖI-Emulsion (W/O-Emulsion, z. B. Butter) handelt es sich um das umgekehrte Prinzip, wobei der Grundcharakter hier durch das Öl bestimmt wird.

Um die dauerhafte Dispergierung einer Flüssigkeit in einer anderen zu erreichen, ist bei Emulsionen im herkömmlichen Sinn der Zusatz einer grenzflächenaktiven Substanz (Emulgator) notwendig. Emulgatoren weisen einen amphiphilen Molekülaufbau auf, bestehend aus einem polaren (hydrophilen) und einem unpolaren (lipophilen) Molekülteil, die räumlich voneinander getrennt sind. In einfachen Emulsionen liegen in der einen Phase feindisperse, von einer Emulgatorhülle umschlossene Tröpfchen der zweiten Phase (Wassertröpfchen in W/O- oder Lipidvesikel in O/W-Emulsionen) vor. Emulgatoren setzen die Grenzflächenspannung zwischen den Phasen herab, indem sie an der Grenzfläche zwischen beiden Flüssigkeiten angeordnet sind. Sie bilden an der Phasengrenze ÖI/Wasser Grenzflächenfilme aus, wodurch dem irreversiblen Zusammenfließen der Tröpfchen entgegengewirkt wird. Zur Stabilisierung von Emulsionen werden häufig Emulgatorgemische verwendet.

Herkömmliche Emulgatoren können entsprechend ihrem hydrophilen Molekülteil in ionische (anionische, kationische und amphotere) und nichtionische untergliedert werden:
- Das wohl bekannteste Beispiel eines anionischen Emulgators ist die Seife, als die man gewöhnlich die wasserlöslichen Natrium- oder Kaliumsalze der gesättigten und ungesättigten höheren Fettsäuren bezeichnet.
- Wichtige Vertreter der kationischen Emulgatoren sind die quartären Ammonium-Verbindungen.
- Der hydrophile Molekülteil nichtionischer Emulgatoren besteht häufig aus Glycerin, Polyglycerin, Sorbitanen, Kohlenhydraten bzw. Polyoxyethylenglykolen und ist meistens über Ester- und Etherbindungen mit dem lipophilen Molekülteil verknüpft. Dieser besteht üblicherweise aus Fettalkoholen, Fettsäuren oder Isofettsäuren.
Durch Variation der Struktur und der Größe des polaren und des unpolaren Molekülteils lassen sich Lipophilie und Hydrophilie von Emulgatoren in weiten Grenzen verändern.

Entscheidend für die Stabilität einer Emulsion ist die richtige Auswahl der Emulgatoren. Dabei sind die Charakteristiken aller im System enthaltenen Stoffe zu berücksichtigen. Betrachtet man z. B. Hautpflegeemulsionen, so führen polare Ölkomponenten und beispielsweise UV-Filter zu Instabilitäten. Neben den Emulgatoren werden daher noch andere Stabilisatoren verwendet, die beispielsweise die Viskosität der Emulsion erhöhen und/oder als Schutzkolloid wirken.

Emulsionen stellen einen wichtigen Produkttyp im Bereich kosmetischer und/oder dermatologischer Zubereitungen dar.

Kosmetische Zubereitungen werden im wesentlichen zur Hautpflege benutzt. Hautpflege im kosmetischen Sinn ist in erster Linie, dass die natürliche Funktion der Haut als Barriere gegen Umwelteinflüsse (z. B. Schmutz, Chemikalien, Mikroorganismen) und gegen den Verlust von körpereigenen Stoffen (z. B. Wasser, natürliche Fette, Elektrolyte) gestärkt oder wiederhergestellt wird. Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Kosmetische Zubereitungen werden auch als Desodorantien verwendet. Solche Formulierungen dienen dazu, Körpergeruch zu beseitigen, der entsteht, wenn der an sich geruchlose frische Schweiß durch Mikroorganismen zersetzt wird.

Medizinische topische Zusammensetzungen enthalten in der Regel ein oder mehrere Arzneistoffe in wirksamer Konzentration. Der Einfachheit halber wird zur eindeutigen Unterscheidung zwischen kosmetischer und medizinischer Anwendung und entsprechenden Produkten auf die gesetzlichen Bestimmungen der Bundesrepublik Deutschland verwiesen (z. B. Kosmetikverordnung, Lebensmittel- und Arzneimittelgesetz).

An sich ist die Verwendung der üblichen Emulgatoren in kosmetischen oder dermatologischen Zubereitungen unbedenklich. Dennoch können Emulgatoren, wie letztlich jede chemische Substanz, im Einzelfalle allergische oder auf Überempfindlichkeit des Anwenders beruhende Reaktionen hervorrufen.

So ist beispielsweise bekannt, dass bestimmte Lichtdermatosen durch gewisse Emulgatoren, aber auch durch verschiedene Fette und gleichzeitige Exposition von Sonnenlicht ausgelöst werden. Solche Lichtdermatosen werden auch "Mallorca-Akne" genannt. Es hat daher nicht an Versuchen gefehlt, die Menge an üblichen Emulgatoren auf ein Minimum, im Idealfall sogar vollständig zu reduzieren.

Eine Reduktion der benötigten Emulgatormenge kann z. B. erreicht werden, wenn ausgenutzt wird, dass feinstverteilte Feststoffteilchen eine zusätzlich stabilisierende Wirkung haben. Dabei kommt es zu einer Anreicherung des festen Stoffes an der Phasengrenze ÖI/Wasser in Form einer Schicht, wodurch ein Zusammenfließen der dispersen Phasen verhindert wird. Von wesentlicher Bedeutung sind dabei nicht die chemischen, sondern die Oberflächeneigenschaften der Feststoffpartikel.

Eine relativ neue technische Entwicklung ist es, kosmetische oder dermatologische Zubereitungen nur durch feinstverteilte Feststoffteilchen zu stabilisieren. Solche "emulgatorfreien" Emulsionen werden nach ihrem Erfinder auch als Pickering-Emulsionen bezeichnet.

Grundlegende Untersuchungen haben dabei gezeigt, dass ein Charakteristikum für eine Pickering-Emulsion ist, dass die Feststoffpartikel an der Grenzfläche zwischen den beiden flüssigen Phasen angeordnet sind und dort sozusagen eine mechanische Barriere gegen die Vereinigung der Flüssigkeitströpfchen bilden.

Die Europäische Offenlegungsschrift 0 686 391 beschreibt darüber hinaus "Emulsionen" vom Typ Wasser-in-ÖI, die frei von oberflächenaktiven Substanzen sind und nur durch Feststoffe stabilisiert werden. Zur Stabilisierung werden hier sphärische Polyalkylsilsesquioxan-Partikel eingesetzt, die einen Durchmesser von 100 nm bis zu 20 µm haben, welche in die Ölphase gegeben werden und diese verdicken. Diese Art von Zubereitungen kann man daher als ÖI-Gele (auch: Oleogele) bezeichnen, in welche Wasser stabil eindispergiert werden kann.

Ferner beschreibt die WO-Schrift WO-98/42301 emulgatorfreie feindisperse Systeme vom Typ Wasser-in-ÖI, welche durch den Zusatz mikronisierter, anorganischer Pigmente stabilisiert werden, die aus der Gruppe der Metalloxide, insbesondere Titandioxid gewählt werden.

In DE 199 39 849 werden nahezu Emulgatorfreie feidisperse Systeme Wasser-in-ÖI beschrieben, die durch modifizierte Schichtsilikate mit amphiphilen Charakter stabilisiert sind.

Allerdings konnte der Stand der Technik nicht den Weg zur vorliegenden Erfindung weisen.

Der Stand der Technik kennt neben den beschriebenen Systemen weitere emulgatorfreie, feindisperse kosmetische oder dermatologische Zubereitungen, die im allgemeinen als Hydrodispersionen bezeichnet werden. Hydrodispersionen stellen Dispersionen einer flüssigen, halbfesten oder festen inneren (diskontinuierlichen) Lipidphase in einer äußeren wäßrigen (kontinuierlichen) Phase dar.

Bei Hydrodispersionen einer flüssigen Lipidphase in einer äußeren wäßrigen Phase kann die Stabilität beispielsweise dadurch gewährleistet werden, dass in der wäßrigen Phase ein Gelgerüst aufgebaut wird (*kohärente Syst*.), in welchem die Lipidtröpfchen stabil suspendiert sind. Die Deutsche Offenlegungsschrift P 44 25 268 A1 beschreibt stabile feindisperse, emulgatorfreie kosmetische oder dermatologische Zubereitungen vom Typ ÖI-in-Wasser, die neben einer ÖI- und einer Wasserphase einen oder mehrere Verdicker aus der Gruppe der Acrylsäurepolymere, Polysaccharide und deren Alkylether enthalten, wobei für diese Verdicker eine Grenzflächenspannungserniedrigung nicht meßbar sein darf.

Basierend auf ähnlichen Hydrodispersionen werden in der Deutschen Offenlegungsschrift P 43 03 983 A1 kosmetische oder dermatologische Lichtschutzformulierungen offenbart, die im wesentlichen frei von Emulgatoren sind, wobei in die Lipidphase der Hydrodispersion anorganische Mikropigmente eingearbeitet sind, die als UV-Filtersubstanzen dienen.

Allerdings können Feststoffe bei der Anwendung entsprechender Zubereitungen auf der Haut einen trockenen und zum Teil stumpfen Eindruck hinterlassen. Bereits Zubereitungen mit einem Pigmentgehalt von 1 Gew.-% erzeugen nach ihrer Anwendung ein stumpfes Gefühl auf der Haut, das mit höheren Pigmentkonzentrationen noch zunimmt. Im Einzelfall können daher pigmenthaltige Zubereitungen sogar nicht vermarktungsfähig sein, da sie vom Verbraucher nicht akzeptiert bzw. negativ beurteilt werden.

Aufgabe war daher, den Nachteilen des Standes der Technik Abhilfe zu schaffen. Insbesondere sollten Zubereitungen zur Verfügung gestellt werden, die trotz einer gelartigen Konsistenz sprühbar sind und auf der Haut keinen trockenen oder stumpfen Eindruck hinterlassen.

Weiterhin war es eine Aufgabe der Erfindung, kosmetische und dermatologische Grundlagen für kosmetische und dermatologische Zubereitungen zu entwickeln, die sich durch gute Hautverträglichkeit auszeichnen.

Ferner war eine Aufgabe der vorliegenden Erfindung, Produkte mit einer möglichst breiten Anwendungsvielfalt zur Verfügung zu stellen. Beispielsweise sollten Grundlagen für Zubereitungsformen wie Reinigungsemulsionen, Gesichts- und Körperpflegezubereitungen oder Desodorantien, aber auch ausgesprochen medizinisch-pharmazeutische Darreichungsformen geschaffen werden, zum Beispiel Zubereitungen gegen Akne und andere Hauterscheinungen.

Eine weitere Aufgabe der vorliegenden Erfindung war, den Stand der Technik um kosmetische oder dermatologische Zubereitungen zu bereichern, in welchen der Einsatz von Emulgatoren herkömmlicher Art auf ein Minimum reduziert ist.

### Erstaunlicherweise werden alle diese Aufgabe gelöst durch:

Kosmetische oder dermatologische Zubereitungen, die feindisperse Systeme vom Typ ÖI-in-Wasser bzw. Silikon-in-Wasser darstellen, enthaltend
1. eine Ölphase und/oder Silikonölphase,
2. eine Wasserphase,
3. mindestens ein unmodifiziertes Schichtsilikat,
4. gegebenenfalls ein oder mehrere modifiziertes Schichtsilikate und
5. mindestens einen Anionischen und/oder nichtionischen Emulgator, sowie gegebenenfalls weitere kosmetische oder pharmazeutische Hilfs-, Zusatzund/oder Wirkstoffe.

In den erfindungsgemäßen Zubereitungen liegen die Schichtsilikate in kolloidal gelöster Form vor.
In kolloidalen Lösungen liegen die feinverteilten Feststoffpartikel, mit Teilchengrößen zwischen 1000 und 1 nm vor.

Eine strenge physikal.-chem. Abgrenzung zwischen eienr kolloiden und einer "echten" Lösung existiert ebenso wenig wie zwischen einer kolloiden Lösung und sich absetzenden Suspensionen. Das Wort "kolloid" bezeichnet jedenfalls keine Stoffeigenschaft, sondern einen Zustand.
Kolloide Lösungen stellen eine spezielle Form der homogenen Suspensionen dar. Eine solche koloidale Lösung wird dadurch erreicht, dass die Schichtsilikate in der Wasserphase unter Einwirkung hoher Scherkräfte dispergiert werden.
Diese Schichtsilikate nehmen dadurch insetiv Fremdmoleküle (hier Wasser) in die Schichtstruktur auf und bilden ein *kohärentes System (Gel)* aus. Bei Belastung brechen diese Schichten unter teilweiser Fragmentierung in kleinere Molekülverbände auf. Dieser Prozess ist reversibel, sobald die Belastung entfällt.

Geeignete Dispergiergeräte sind u.a. unter der Bezeichnung Ultra-Turrax® (Firma IKA) erhältlich und schaffen Endfeinheiten der Partikel von bis zu 5 µm.

Die erfindungsgemäßen Zubereitungen sind trotz ihrer Gelstruktur sprühbare Formulierungen, die eine Viskosität und/oder Fließgrenze von über 2500 mPas (Messung mit Viskotester VT-02 der Fa. Haake, Drehkörper 1,Messwert nach 30s, Skala 1) bis zur cremeartigen Konsistenz aufweisen. Normalerweise müssen sprühbare Formulierungen eine Viskosität von unter 1000 mPas, besser noch unter 500 mPas (oder wasserdünn) aufweisen, um mit normalen Sprühköpfen versprühbar zu sein.

Die erfindungsgemäßen Zubereitungen sind durch herkömmliche Sprühköpfe sprühbar, da durch die beim Druck auf den Sprühkopf (Förderpumpe) auftretenden Scherkräfte das Schichtsilikatgerüst kurzfristig und reversibel zusammenbricht und die Zubereitung damit dünnflüssig und sprühbar wird (Thixotropie).

In das aus Schichtsilikat und Wasser gebildete Gelgerüst sind in den erfindungsgemäßen Zubereitungen die Lipidtröpfchen stabil suspendiert.

Die Stabilität von kolloidalen Lösungen wird stark durch die Oberflächenladungen der Partikel, ihre elektrostatischen Wechselwirkungen sowie durch Leitfähigkeit u. pH-Wert der äußeren Phase bestimmt. Das Über- od. Unterschreiten eines bestimmten pH-Wertes (isoelektrischer Punkt) führt zum Ausflocken (Koagulation) der Dispersion, ebenso wie das Überschreiten einer bestimmten Leitfähigkeit.

Die erfindungsgemäßen Zubereitungen stellen Abmischungen aus Ölen bzw. öllöslichen Substanzen und Wasser bzw. wasserlöslichen Komponenten dar, die durch den Zusatz von Schichtsilikaten und gegebenenfalls modifizierten Schichtsilikatpartikeln stabilisiert werden und nur geringe Mengen an herkömmlichen Emulgatoren enthalten müssen.

Die erfindungsgemäßen Zubereitungen sind in jeglicher Hinsicht überaus befriedigende Präparate, die erstaunlicherweise hervorragende kosmetische Eigenschaften zeigen, auf der Haut keinen trockenen oder stumpfen Eindruck hinterlassen und eine ausgezeichnete Hautverträglichkeit aufweisen. Insbesondere die Bildung von röllchenartigen Partikeln, die beim Aneinanderreihen von mit herkömmlichen kosmetischen Zubereitung behandelten Hautarealen entstehen, ist bei den erfindungsgemäßen Zubereitungen nicht erkennbar.

Ein weiterer Vorteil ist, dass die Struktur in der Zubereitung erhalten bleibt und nach der Abfüllung stabil ist. Auch während der Lagerung oder des Transports zum Verwender tritt keine Separation der Emulsionen und den darin enthaltenen Wirkstoffen und/oder Pigmenten ein. Ein Aufschwimmen oder Absinken von Partikeln ist ebenfalls, bei Bedingungen die für die Lagerung von kosmetischen Zubereitungen üblich sind, nicht zu beobachten.

In Zubereitungen des Standes der Technik wird die Separation und das Aufschwimmen (Floating) oft über eine Erhöhung der Viskosität erreicht, wodurch der Vorgang des sogenannten Floating dann jedoch nur verlangsamt wird, aber nicht verhindert werden kann. Bei zu hoher Viskosität ist jedoch eine Sprühbarkeit nicht mehr gegeben.

Bei den erfindungsgemäßen Zubereitungen wird das Floating durch das Gelgerüst unterdrückt.

Bei Verwendung des geeigneten Schicktsilikats wird, durch den Aufbau einer hohen Fließgrenze, das Floating verhindert. Während der Lagerung und des Transports der abgefüllten Zubereitungen kommt es zu keiner Veränderung der Textur oder sogenannter Nachverdickung. Durch die starke Viskositätserniedrigung, die bei einer Scherbeanspruchung auftritt, lassen sich die erfindungsgemäßen Formulierungen jedoch versprühen.

Zubereitungen mit optimaler Textur zeigen nur unter Scherbeanspruchung (beim Pumpen) eine Viskositätserniedrigung (strukturviskoses Verhalten).
Eine hohe Scherstabilität ist wichtig, um auch nach mehrmaligem Pumpen der Zubereitung eine ausreichend hohe Viskosität und gute Stabilität zu garantieren.
Nach Beendigung des Pumpvorgangs bzw. Verteilvorgangs auf der Haut soll die Zubereitungen im Ruhezustand wieder ,regenerieren' und so die notwendige Viskosität aufbauen, um die Wirkstoffe länger auf der Haut halten zu können, und eine verlängerte Wirkung zu ermöglichen.

### Schichtsilikate

Silikate sind Salze und Ester (Kieselsäureester) der Orthokieselsäure [Si(OH)₄] und deren Kondensationsprodukte. Die Silikate sind nicht nur die artenreichste Klasse der Mineralien, sondern auch geologisch und technisch außerordentlich wichtig. Über 80 % der Erdkruste bestehen aus Silikaten. Schichtsilikate (Phyllosilikate, Blattsilikate) sind (idealerweise) Silikat-Strukturen mit zweidimensional unendlichen Schichten aus [SiO₄]⁴⁻-Tetraedern, wobei jedes Tetraeder über 3 Brücken-Sauerstoffe mit Nachbar-Tetraedern verbunden ist.

Chemische Formeln lassen sich für Schichtsilikate nur angenähert aufstellen, da sie ein großes lonenaustausch-Vermögen besitzen und Silizium gegen Aluminium und dieses wiederum gegen Magnesium, Fe²⁺, Fe³⁺, Zn und dergleichen ausgetauscht werden kann. Die daraus möglicherweise resultierende negative Ladung der Schichten wird in der Regel durch Kationen, insbesondere durch Na⁺ und Ca²⁺ in Zwischenschicht-Positionen ausgeglichen.

Schichtsilikate können durch reversible Einlagerung von Wasser (in der 2- bis 7-fachen Menge) und anderen Substanzen wie z. B. Alkoholen, Glykolen und dergleichen mehr aufquellen. Ihre Verwendung als Verdickungsmittel in kosmetischen Mitteln ist dementsprechend an sich bekannt. Allerdings konnte der Stand der Technik nicht den Weg zur vorliegenden Erfindung weisen.

Vorteilhafte Schichtsilikate im Sinne der vorliegenden Erfindung sind beispielsweise solche, deren größte Ausdehnungsrichtung im unmodifizierten und ungequollenen Zustand im Mittel eine Länge von weniger als 10 µm hat.

Die in der Natur vorkommenden Schichtsilikate müssen vor der Verwendung aufbereitet werden. Unter Aufbereitung werden u.a. die Reinigung durch Waschen und das Mahlen verstanden. Insbesondere vorteilhaft ist auch die isomorphe Substitution von der in den natürlichen Schichtsilikaten vorkommenden Kationen mit hoher Valenz durch Kationen geringerer Valenz. Dazu können die natürlichen Schichtsilikate mit einem hohen Überschuss an Natriumsalzen, insbesondere Natriumcarbonat, behandelt, was nicht nur zu einer Reduktion der Kalzium- und Magnesium-Ionen führt, sondern auch die Zwischenlagen-Protonen-Bindungsstellen (engl. interlayer protonic sites) absättigt und gebundene natürliche organische Säuren ersetzt.

Beispielsweise können die mittleren Ausdehnungen der verwendeten aufbereiteten Schichtsilikatpartikel bei 1000 nm x 100 nm x 1 nm und darunter liegen. Die effektive Größe der aufbereiteten Schichtsilikatpartikel in einer kosmetischen oder dermatologischen Formulierung hängt selbstverständlich von der Menge an eingelagerten Substanzen ab.

Vorteilhafte Schichtsilikate im Sinne der vorliegenden Erfindung sind beispielsweise aufbereiteten Smektite (Smectite).
Smektite sind stets sehr feinkörnige (meist < 2 mm), überwiegend als lamellenförmige, moosartige oder kugelförmige Aggregate vorkommende Dreischicht-Tonminerale (2:1-Schichtsilikate), in denen eine zentrale Schicht aus oktaedrisch koordinierten Kationen sandwichartig von 2 Schichten aus [(Si,Al)O₄]-Tetraedern umgeben ist. Smektite werden idealisiert durch die folgende Strukturformel beschrieben, worin weiß ausgefüllte Kreise Silizium- und/oder Aluminiumatome, hellgrau ausgefüllte Kreise Sauerstoffatome, dunkelgrau ausgefüllte Kreise Wasserstoffatome und schwarz ausgefüllte Kreise Aluminium-, Magnesium-, Eisenatome und/oder weitere Austauschkationen darstellen:

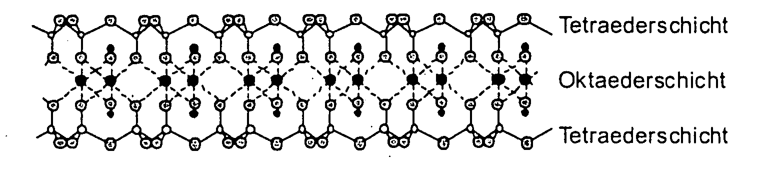

Vorteilhafte aufbereiteten Smektite sind z. B. aufbereiteten **Montmorillonlte**. Montmorillonite werden durch die angenäherte chemische Formel Al₂[(OH)₂/Si₄O₁₀] · n H₂O bzw. Al₂O₃ · 4 SiO₂ · H₂O · n H₂O beschrieben und stellen zu den dioktaedrischen Smektiten gehörende Tonmineralien dar.

Vorteilhaft im Sinne der vorliegenden Erfindung sind ferner aufbereitete **Bentonite**. Bentonite sind Tone und Gesteine, die Smektite, vor allem Montmorillonit, als Hauptminerale enthalten. Die "Roh"-Bentonite sind entweder die nicht quellfähigen Calcium-Bentonite (in Großbritannien als Fuller-Erden bezeichnet) oder die quellfähigen Natrium-Bentonite (auch: Wyoming-Bentonite).
Durch teilweisen isomorphen Austausch von Ca-Ionen im Ca-Bentonit durch Na-Ionen, können Fuller-Erden quellfähig gemacht werden.

Hectorite sind ebenfalls ein zu den Smektiten gehörendes, dem Montmorillonit ähnliches, monoklines Tonmineral, mit der Formel M_{0.3}⁺(Mg_{2.7}Li_{0.3})[Si₄O₁₀(OH)₂], mit M⁺ meist = Na⁺. Besonders vorteilhaft im Sinne der vorliegenden Erfindung sind die aufbereiteten Hektorite.
Insbesondere Vorteilhaft ist die Verwendung von synthetischen Natrium Magnesium Silikaten (INCI Name: Sodium Magnesium Silicate) vom Typ Optigel SH® und vom Typ Laponite XLG®.

Saponit ist ein zu den Smektiten gehörendes, quellfähiges Magnesiumaluminiumsilikat in dem die Oktaeder-Schichten in der Struktur fast ausschließlich von Mg-, Fe²⁺- und Fe³⁺- Ionen besetzt sind. Eine negative Ladung von x ≤ 0,6 pro O₁₀(OH)₂ in den Tetraederschichten wird durch Kationen zwischen den Schichtpaketen ausgeglichen. (Formel Mg₃[(Si₄ -ₓAlₓ)O₁₀/(OH)₂]Eₓ⁺ und *Eisen-Saponit* (Fe²⁺ statt Mg); für x = 0,33 kann z. B. Eₓ⁺=Na^{0,33+} stehen.)
Insbesondere vorteilhaft ist die Verwendung von Magnesiumaluminiumsilikaten (INCI Name: Magnesium Aluminium Silicate) z.B. vom Typ VEEGUM®.(natürlichen Ursprungs).

Ganz besonders Schichtsilikate vom Typ Laponite XLG ® (Fa. Solvay) zeigen in einer Konzentration von 1 bis 5 Gew.-% - bezogen auf das Gesamtgewicht der Zubereitungen - und in Kombination mit nichtionischen und anionischen Emulgatoren das gewünschte Gelbildungsverhalten.

Die durch die Schichtsilikate ausgelöste Gelbildung wird durch Zusatz von Elektrolyten unterstützt, jedoch kann bei zu hohen Elektrolytgehalten eine Ausflockung auftreten.

Erfindungsgemäß ist insbesondere auch die Verwendung von synthetischen Schichtsilikaten, bzw. sehr reinen, aufbereiteten natürlichen Schichtsilikaten, da diese auch bei geringer Scherung und ohne Einwirkung von Hitze zu rascher Gelbildung neigen.

### Modifizierte Schichtsilikate

Modifizierte Schichtsilikate im Sinne der vorliegenden Erfindung sind Schichtsilikate, insbesondere die bereits genannten Schichtsilikattypen, deren Organophilie (auch: Lipophilie) - beispielsweise durch Umsetzung mit quarternären Ammonium-Verbindungen - erhöht wurde. Solche Schichtsilikate werden auch als organophile Schichtsilikate bezeichnet.

Besonders vorteilhaft im Sinne der vorliegenden Erfindung sind sogenannte Bentone, d. h. organische Derivate von Montmorilloniten (bzw. Bentoniten) und/oder Hectoriten, die durch lonenaustausch-Reaktionen mit Alkylammonium-Basen hergestellt werden.

Vorteilhafte modifizierte Schichtsilikate im Sinne der vorliegenden Erfindung sind beispielsweise durch Umsetzung von Schichtsilikaten mit Quaternium-18 erhältlich. Quaternium-18 ist eine Mischung von quaternären Ammoniumchloridsalzen, welche durch die folgende Strukturformel beschrieben werden: 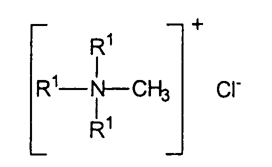 worin
die Reste R¹ unabhängig voneinander gewählt werden aus der Gruppe Methyl und hydrierte Talgreste mit einer Kettenlänge von 12 bis 20 Kohlenstoffatomen.

Erfindungsgemäß besonders bevorzugt sind Stearalkoniumhektorit, ein Reaktionsprodukt aus Hektorit und Stearalkoniumchlorid (Benzyldimethylstearylammoniumchlorid), und Quaternium-18 Hektorit, ein Reaktionsprodukt aus Hektorit und Quaternium-18, welche z. B. unter den Handelsbezeichnungen Bentone 27 und Bentone 38 bei Nordmann & Rassmann erhältlich sind.

Der Vorteil in der Verwendung von modifizierten Schichtsilikaten in erfindungsgemäßen Zubereitungen, liegt in einer Reduktion der Menge herkömmlicher Emulgatoren, die die Stabilität der O/W-Emulsion hervorrufen.

Die Gesamtmenge an einem oder mehreren Schichtsilikaten und/oder modifizierten Schichtsilikaten in den fertigen kosmetischen oder dermatologischen Zubereitungen wird vorteilhaft aus dem Bereich von 0,05 bis 20,0 Gew.-%, bevorzugt 0,1 bis 5,0 Gew.-% gewählt, bezogen auf das Gesamtgewicht der Zubereitungen.

Obwohl es besonders bevorzugt ist, die erfindungsgemäßen Zubereitungen nur durch den Zusatz von einem oder mehreren Schichtsilikaten und/oder modifizierten Schichtsilikaten zu stabilisieren, kann es ferner vorteilhaft sein, weitere amphiphile Pigmente einzusetzen, welche zur Verbesserung des Hautgefühls, der Einfärbung der Zubereitung und des UV-Schutzes der Zubereitung und insbesondere der Haut dienen.

Solche Pigmente sind beispielsweise mikronisierte, anorganische Pigmente, die gewählt werden aus der Gruppe der amphiphilen Metalloxide, insbesondere aus der Gruppe Titandioxid, Zinkoxid, Siliciumdioxid bzw. Silicate (z. B. Talkum), wobei die Metalloxide sowohl einzeln als auch im Gemisch vorliegen können. Dabei ist es im wesentlichen unerheblich, in welcher der gegebenenfalls natürlich vorkommenden Modifikationen die verwendeten amphiphilen Metalloxide vorliegen.

Vorteilhaft ist es, den mittleren Partikeldurchmesser, der zur Kombination mit Schichtsilikaten und/oder modifizierten Schichtsilikaten verwendeten Pigmente zwischen 1 nm und 200 nm, besonders vorteilhaft zwischen 5 nm und 100 nm zu wählen.

Vorteilhaft im Sinne der vorliegenden Erfindung ist es, die erfindungsgemäßen modifizierten Schichtsilikate mit unbehandelten, nahezu reinen Pigmentpartikeln zu kombinieren, insbesondere mit solchen, welche auch als Farbstoff in der Lebensmittelindustrie und/oder als Absorber von UV-Strahlung in Sonnenschutzmitteln verwendet werden können. Vorteilhaft sind beispielsweise die bei der Firma Merck erhältlichen Zinkoxid-Pigmente sowie solche, die unter den Handelsbezeichnungen Zinkoxid neutral bei Haarmann & Reimer oder NanoX von der Harcros Chemical Group erhältlich sind.

Erfindungsgemäß vorteilhaft ist ferner die Kombination von Schichtsilikaten und/oder modifizierten Schichtsilikaten mit anorganischen Pigmenten, die oberflächlich wasserabweisend behandelt ("gecoatet") sind, wobei gleichzeitig ein amphiphiler Charakter dieser Pigmente gebildet werden bzw. erhalten bleiben soll. Diese Oberflächenbehandlung kann darin bestehen, dass die Pigmente nach an sich bekannten Verfahren mit einer dünnen hydrophoben Schicht versehen werden.

Vorteilhaft in allen vorgenannten Fällen ist es, die Gesamtkonzentration aller Pigmente größer als 0,05 Gew.-%, besonders vorteilhaft zwischen 0,05 Gew.-% und 30 Gew.-% bezogen auf das Gesamtgewicht der Zubereitungen zu wählen, wobei die Konzentration an Schichtsilikaten und/oder modifizierten Schichtsilikaten - ebenfalls bezogen auf das Gesamtgewicht der Zubereitungen - im Sinne der vorliegenden Erfindung vorzugsweise aus dem Bereich 0,01 Gew.-% bis 20 Gew.-%, vorteilhaft 0,5 Gew.-% bis 10 Gew.-% zu wählen ist.

### Emulgatoren

Vorteilhaft wird oder werden der oder die O/W-Emulgatoren gewählt aus der Gruppe
- der Fettalkoholethoxylate der allgemeinen Formel R-O-(-CH₂-CH₂-O-)ₙ-H, wobei R einen verzweigten oder unverzweigten Alkyl-, Aryl- oder Alkenylrest und n eine Zahl von 10 bis 50 darstellen
- der ethoxylierten Wollwachsalkohole,
- der Polyethylenglycolether der allgemeinen Formel R-O-(-CH₂-CH₂-O-)ₙ-R', wobei R und R' unabhängig voneinander verzweigte oder unverzweigte Alkyl- oder Alkenylreste und n eine Zahl von 10 bis 80 darstellen
- der Fettsäureethoxylate der allgemeinen Formel
   R-COO-(-CH₂-CH₂-O-)ₙ -H, wobei R einen verzweigten oder unverzweigten Alkyloder Alkenylrest und n eine Zahl von 10 bis 40 darstellen,
- der veretherten Fettsäureethoxylate der allgemeinen Formel
   R-COO-(-CH₂-CH₂-O-)ₙ -R', wobei R und R' unabhängig voneinander verzweigte oder unverzweigte Alkyl- oder Alkenylreste und n eine Zahl von 10 bis 80 darstellen,
- der veresterten Fettsäureethoxylate der allgemeinen Formel
   R-COO-(-CH₂-CH₂-O-)ₙ -C(O)-R', wobei R und R' unabhängig voneinander verzweigte oder unverzweigte Alkyl- oder Alkenylreste und n eine Zahl von 10 bis 80 darstellen,
- der Polyethylenglycolglycerinfettsäureester gesättigter und/oder ungesättigter, verzweigter und/oder unverzweigter Fettsäuren und einem Ethoxylierungsgrad zwischen 3 und 50,
- der ethoxylierten Sorbitanester mit einem Ethoxylierungsgrad von 3 bis 100
- der Cholesterinethoxylate mit einem Ethoxylierungsgrad zwischen 3 und 50,
- der ethoxylierten Triglyceride mit einem Ethoxylierungsgrad zwischen 3 und 150,
- der Alkylethercarbonsäuren der allgemeinen Formel
   R-O-(-CH₂-CH₂-O-)ₙ-CH₂-COOH bzw. deren kosmetisch oder pharmazeutisch akzeptablen Salze, wobei R einen verzweigten oder unverzweigten Alkyl- oder Alkenylrest mit 5 - 30 C-Atomen und n eine Zahl von 5 bis 30 darstellen,
- der Polyoxyethylensorbitolfettsäureester, basierend auf verzweigten oder unverzweigten Alkan- oder Alkensäuren und einen Ethoxylierungsgrad von 5 bis 100 aufweisend, beispielsweise vom Sorbeth-Typ,
- der Alkylethersulfate bzw. die diesen Sulfaten zugrundeliegenden Säuren der allgemeinen Formel R-O-(-CH₂-CH₂-O-)ₙ-SO₃-H mit kosmetisch oder pharmazeutisch akzeptablen Kationen, wobei R einen verzweigten oder unverzweigten Alkyloder Alkenylrest mit 5 - 30 C-Atomen und n eine Zahl von 1 bis 50 darstellen.
- der Fettalkoholpropoxylate der allgemeinen Formel
   R-O-(-CH₂-CH(CH₃)-O-)ₙ-H, wobei R einen verzweigten oder unverzweigten Alkyloder Alkenylrest und n eine Zahl von 10 bis 80 darstellen,
- der Polypropylenglycolether der allgemeinen Formel
   R-O-(-CH₂-CH(CH₃)-O-)ₙ-R', wobei R und R' unabhängig voneinander verzweigte oder unverzweigte Alkyl- oder Alkenylreste und n eine Zahl von 10 bis 80 darstellen
- der propoxylierten Wollwachsalkohole,
- der veretherten Fettsäurepropoxylate der allgemeinen Formel
   R-COO-(-CH₂-CH(CH₃)-O-)ₙ-R', wobei R und R' unabhängig voneinander verzweigte oder unverzweigte Alkyl- oder Alkenylreste und n eine Zahl von 10 bis 80 darstellen,
- der veresterten Fettsäurepropoxylate der allgemeinen Formel
   R-COO-(-CH₂-CH(CH₃)-O-)ₙ-C(O)-R', wobei R und R' unabhängig voneinander verzweigte oder unverzweigte Alkyl- oder Alkenylreste und n eine Zahl von 10 bis 80 darstellen,
- der Fettsäurepropoxylate der allgemeinen Formel
   R-COO-(-CH₂-CH(CH₃)-O-)ₙ-H, wobei R einen verzweigten oder unverzweigten Alkyl- oder Alkenylrest und n eine Zahl von 10 bis 80 darstellen,
- der Polypropylenglycolglycerinfettsäureester gesättigter und/oder ungesättigter, verzweigter und/oder unverzweigter Fettsäuren und einem Propoxylierungsgrad zwischen 3 und 80
- der propoxylierten Sorbitanester mit einem Propoxylierungsgrad von 3 bis 100
- der Cholesterinpropoxylate mit einem Propoxylierungsgrad von 3 bis 100
- der propoxylierten Triglyceride mit einem Propoxylierungsgrad von 3 bis 100
- der Alkylethercarbonsäuren der allgemeinen Formel
   R-O-(-CH₂-CH(CH₃)O-)ₙ-CH₂-COOH bzw. deren kosmetisch oder pharmazeutisch akzeptablen Salze, wobei R einen verzweigten oder unverzweigten Alkyl- oder Alkenylrest und n eine Zahl von 3 bis 50 darstellen,
- der Alkylethersulfate bzw. die diesen Sulfaten zugrundeliegenden Säuren der allgemeinen Formel R-O-(-CH₂-CH(CH₃)-O-)n-SO₃-H mit kosmetisch oder pharmazeutisch akzeptablen Kationen, wobei R einen verzweigten oder unverzweigten Alkyl- oder Alkenylrest mit 5 - 30 C-Atomen und n eine Zahl von 1 bis 50 darstellen,
- der Fettalkoholethoxylate/propoxylate der allgemeinen Formel
   R-O-Xₙ-Yₘ-H, wobei R einen verzweigten oder unverzweigten Alkyl- oder Alkenylrest darstellen, wobei X und Y nicht identisch sind und jeweils entweder eine Oxyethylengruppe oder eine Oxypropylengruppe und n und m unabhängig voneinander Zahlen von 5 bis 50 darstellen,
- der Polypropylenglycolether der allgemeinen Formel
   R-O-Xₙ-Yₘ-R', wobei R und R' unabhängig voneinander verzweigte oder unverzweigte Alkyl- oder Alkenylreste darstellen, wobei X und Y nicht identisch sind und jeweils entweder eine Oxyethylengruppe oder eine Oxypropylengruppe und n und m unabhängig voneinander Zahlen von 5 bis 100 darstellen,
- der veretherten Fettsäurepropoxylate der allgemeinen Formel
   R-COO-Xₙ-Yₘ-R', wobei R und R' unabhängig voneinander verzweigte oder unverzweigte Alkyl- oder Alkenylreste darstellen, wobei X und Y nicht identisch sind und jeweils entweder eine Oxyethylengruppe oder eine Oxypropylengruppe und n und m unabhängig voneinander Zahlen von 5 bis 100 darstellen,
- der Fettsäureethoxylate/propoxylate der allgemeinen Formel
   R-COO-Xₙ-Yₘ-H, wobei R einen verzweigten oder unverzweigten Alkyl- oder Alkenylrest, wobei X und Y nicht identisch sind und jeweils entweder eine Oxyethylengruppe oder eine Oxypropylengruppe und n und m unabhängig voneinander Zahlen von 5 bis 50 darstellen.

Vorteilhaft in allen vorgenannten Fällen ist es, die Gesamtkonzentration aller Emulgatoren größer als 1,0 Gew.-%, besonders vorteilhaft zwischen 1,0 Gew.-% und 5,5 Gew.-% bezogen auf das Gesamtgewicht der Zubereitungen zu wählen.

Es ist erfindungsgemäß besonders vorteilhaft, wenn die Zubereitungen deutlich weniger als 5,5 Gew.-% eines oder mehrerer Emulgatoren enthalten. Ganz besonders bevorzugt sind erfindungsgemäße Zubereitungen, welche 0,001 bis 3,5 Gew.-% eines oder mehrerer Emulgatoren enthalten.

### Ölkomponenten

Eine gegebenenfalls gewünschte Ölkomponente der kosmetischen oder dermatologischen Reinigungszubereitungen - beispielsweise in Form von Reinigungsemulsionen - im Sinne der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z. B. Jojobaöl.

Ferner kann die Ölkomponente vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 bis 18 C-Atomen. Die Fettsäuretriglyceride können beispielsweise vorteilhaft gewählt werden aus der Gruppe der synthetischen, halbsynthetischen und natürlichen Öle, z. B. Olivenöl, Sonnenblumenöl, Sojaöl, Erdnußöl, Rapsöl, Mandelöl, Palmöl, Kokosöl, Palmkernöl und dergleichen mehr.

Auch beliebige Abmischungen solcher ÖI- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölkomponente gewählt aus der Gruppe 2-Ethylhexylisostearat, Octyldodecanol, Isotridecylisononanoat, Isoeicosan, 2-Ethylhexylcocoat, C12-15-Alkylbenzoat, Capryl-Caprinsäure-triglycerid, Dicaprylylether.

Besonders vorteilhaft sind Mischungen aus C12-15-Alkylbenzoat und 2-Ethylhexylisostearat, Mischungen aus C12-15-Alkylbenzoat und Isotridecylisononanoat sowie Mischungen aus C12-15-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölkomponente ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden.

Vorteilhaft wird Cyclomethicon (Octamethylcyclotetrasiloxan) als erfindungsgemäß zu verwendendes Silikonöl eingesetzt. Aber auch andere Silikonöle sind vorteilhaft im Sinne der vorliegenden Erfindung zu verwenden, beispielsweise Hexamethylcyclotrisiloxan, Polydimethylsiloxan, Poly(methylphenylsiloxan).

Besonders vorteilhaft sind ferner Mischungen aus Cyclomethicon und Isotridecylisononanoat, aus Cyclomethicon und 2-Ethylhexylisostearat.

Die Ölkomponente wird ferner vorteilhaft aus der Gruppe der Phospholipide gewählt. Die Phospholipide sind Phosphorsäureester acylierter Glycerine. Von größter Bedeutung unter den Phosphatidylcholinen sind beispielsweise die Lecithine, welche sich durch die allgemeine Struktur 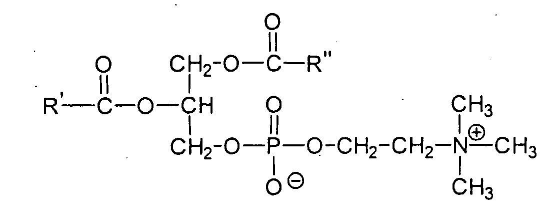 auszeichnen, wobei R' und R" typischerweise unverzweigte aliphatische Reste mit 15 oder 17 Kohlenstoffatomen und bis zu 4 cis-Doppelbindungen darstellen.

Die erfindungsgemäßen Emulsionen oder Gele können als Grundlage für kosmetische oder dermatologische Formulierungen dienen. Diese können wie üblich zusammengesetzt sein und beispielsweise zur Behandlung und zur Pflege der Haut, der Nägel, als Lippenpflegeprodukt, als Deoprodukt und als Schmink- bzw. Abschminkprodukt in der dekorativen Kosmetik oder als Lichtschutzpräparat dienen. Zur Anwendung werden die erfindungsgemäßen kosmetischen und dermatologischen Zubereitungen in der für Kosmetika üblichen Weise auf die Haut in ausreichender Menge aufgebracht.

Entsprechend können kosmetische oder topische dermatologische Zusammensetzungen im Sinne der vorliegenden Erfindung, je nach ihrem Aufbau, beispielsweise verwendet werden als Hautschutzcreme, Reinigungsmilch, Sonnenschutzlotion, Nährcreme, Tagesoder Nachtcreme usw. Es ist gegebenenfalls möglich und vorteilhaft, die erfindungsgemäßen Zusammensetzungen als Grundlage für pharmazeutische Formulierungen zu verwenden.

Die erfindungsgemäßen kosmetischen und dermatologischen Zubereitungen können kosmetische Hilfsstoffe enthalten, wie sie üblicherweise in solchen Zubereitungen verwendet werden, z. B. Konservierungsmittel, Bakterizide, Parfüme, Substanzen zum Verhindern des Schäumens, Farbstoffe, Pigmente, die eine färbende Wirkung haben, Verdickungsmittel, weichmachende, anfeuchtende und/oder feuchthaltende Substanzen, Fette, Öle, Wachse oder andere übliche Bestandteile einer kosmetischen oder dermatologischen Formulierung wie Alkohole, Polyole, Polymere, Schaumstabilisatoren, Elektrolyte, organische Lösemittel oder Silikonderivate.

Eine erstaunliche Eigenschaft der erfindungsgemäßen Zubereitungen ist, dass diese sehr gute Vehikel für kosmetische oder dermatologische Wirkstoffe in die Haut sind, wobei vorteilhafte Wirkstoffe Antioxidantien sind, welche die Haut vor oxidativer Beanspruchung schützen können.

Erfindungsgemäß enthalten die Zubereitungen vorteilhaft eines oder mehrere Antioxidantien. Als günstige, aber dennoch fakultativ zu verwendende Antioxidantien können alle für kosmetische und/oder dermatologische Anwendungen geeigneten oder gebräuchlichen Antioxidantien verwendet werden. Es ist dabei vorteilhaft, Antioxidantien als einzige Wirkstoffklasse zu verwenden, etwa dann, wenn eine kosmetische oder dermatologische Anwendung im Vordergrund steht, wie z. B. die Bekämpfung der oxidativen Beanspruchung der Haut. Es ist aber auch günstig, die erfindungsgemäßen Zubereitungen mit einem Gehalt an einem oder mehreren Antioxidantien zu versehen, wenn die Zubereitungen einem anderen Zweck dienen sollen, z. B. als Desodorantien oder Sonnenschutzmittel.

Besonders vorteilhaft werden die Antioxidantien gewählt aus der Gruppe bestehend aus Aminosäuren (z. B. Glycin, Histidin, Tyrosin, Tryptophan) und deren Derivate, Imidazole (z. B. Urocaninsäure) und deren Derivate, Peptide wie D,L-Carnosin, D-Carnosin, L-Carnosin und deren Derivate (z. B. Anserin), Carotinoide, Carotine (z. B. α-Carotin, β-Carotin, Lycopin) und deren Derivate, Chlorogensäure und deren Derivate, Liponsäure und deren Derivate (z. B. Dihydroliponsäure), Aurothioglucose, Propylthiouracil und andere Thiole (z. B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide; Nukleoside und Salze) sowie Sulfoximinverbindungen (z. B. Buthioninsulfoximine, Homocysteinsulfoximin, Buthioninsulfone, Penta-, Hexa-, Heptathioninsulfoximin) in sehr geringen verträglichen Dosierungen (z. B. pmol bis µmol/kg), ferner (Metall)-Chelatoren (z. B. α-Hydroxyfettsäuren, Palmitinsäure, Phytinsäure, Lactoferrin), α-Hydroxysäuren (z. B. Citronensäure, Milchsäure, Apfelsäure), Huminsäure, Gallensäure, Gallenextrakte, Bilirubin, Biliverdin, EDTA, EGTA und deren Derivate, ungesättigte Fettsäuren und deren Derivate (z. B. γ-Linolensäure, Linolsäure, Ölsäure), Folsäure und deren Derivate, Ubichinon und Ubichinol und deren Derivate, Vitamin C und Derivate (z. B. Ascorbylpalmitat, Mg-Ascorbylphosphat, Ascorbylacetat), Tocopherole und Derivate (z. B. Vitamin-E-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Zink und dessen Derivate (z. B. ZnO, ZnSO₄), Selen und dessen Derivate (z. B. Selenmethionin), Stilbene und deren Derivate (z. B. Stilbenoxid, Trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

Die Menge der vorgenannten Antioxidantien (eine oder mehrere Verbindungen) in den erfindungsgemäßen Zubereitungen beträgt vorzugsweise 0,001 bis 30 Gew.-%, besonders bevorzugt 0,05 - 20 Gew.-%, insbesondere 0,1 - 10 Gew.-%, bezogen auf das Gesamtgewicht der Zubereitung.

Sofern Vitamin E und/oder dessen Derivate das oder die Antioxidantien darstellen, ist es vorteilhaft, deren jeweilige Konzentrationen aus dem Bereich von 0,001 - 10 Gew.-%, bezogen auf das Gesamtgewicht der Formulierung, zu wählen.

Sofern Vitamin A, bzw. Vitamin-A-Derivate, bzw. Carotine bzw. deren Derivate das oder die Antioxidantien darstellen, ist es vorteilhaft, deren jeweilige Konzentrationen aus dem Bereich von 0,001 - 10 Gew.-%, bezogen auf das Gesamtgewicht der Formulierung, zu wählen.

Erfindungsgemäß können die Wirkstoffe (eine oder mehrere Verbindungen) auch sehr vorteilhaft gewählt werden aus der Gruppe der lipophilen Wirkstoffe, insbesondere aus folgender Gruppe:

Acetylsalicylsäure, Atropin, Azulen, Hydrocortison und dessen Derivaten, z. B. Hydrocortison-17-valerat, Vitamine, z. B. Ascorbinsäure und deren Derivate, Vitamine der B- und D-Reihe, sehr günstig das Vitamin B₁, das Vitamin B₁₂ das Vitamin D₁, aber auch Bisabolol, ungesättigte Fettsäuren, namentlich die essentiellen Fettsäuren (oft auch Vitamin F genannt), insbesondere die gamma-Linolensäure, Ölsäure, Eicosapentaënsäure, Docosahexaensäure und deren Derivate, Chloramphenicol, Coffein, Prostaglandine, Thymol, Campher, Extrakte oder andere Produkte pflanzlicher und tierischer Herkunft, z. B. Nachtkerzenöl, Borretschöl oder Johannisbeerkernöl, Fischöle, Lebertran aber auch Ceramide und ceramidähnliche Verbindungen und so weiter.

Vorteilhaft ist es auch, die Wirkstoffe aus der Gruppe der rückfettenden Substanzen zu wählen, beispielsweise Purcellinöl, Eucerit® und Neocerit®.

Die Liste der genannten Wirkstoffe bzw. Wirkstoffkombinationen, die in den erfindungsgemäßen Emulsionen oder Gele verwendet werden können, soll selbstverständlich nicht limitierend sein.

Günstig sind auch solche kosmetischen und dermatologischen Zubereitungen, die in der Form eines Sonnenschutzmittels vorliegen. Vorzugsweise enthalten diese neben den erfindungsgemäßen Wirkstoffkombinationen zusätzlich mindestens eine UV-A-Filtersubstanz und/oder mindestens eine UV-B-Filtersubstanz. Solche Formulierungen können, obgleich nicht notwendig, gegebenenfalls auch ein oder mehrere anorganische Pigmente als UV-Filtersubstanzen enthalten.

Bevorzugt sind anorganische Pigmente auf Basis von Metalloxiden und/oder anderen in Wasser schwerlöslichen oder unlöslichen Metallverbindungen enthalten, insbesondere der Oxide des Titans (TiO₂), Zinks (ZnO), Eisens (z. B. Fe₂O₃), Zirkoniums (ZrO₂), Siliciums (SiO₂), Mangans (z. B. MnO), Aluminiums (Al₂O₃), Cers (z. B. Ce₂O₃), Mischoxiden der entsprechenden Metalle sowie Abmischungen aus solchen Oxiden.

Auch ein zusätzlicher Gehalt an stabilisierend wirkenden Titandioxid- und/oder Zinkoxidpartikeln kann selbstverständlich vorteilhaft sein, ist aber im Sinne der vorliegenden Erfindung nicht notwendig.

Es ist auch vorteilhaft im Sinne der vorliegenden Erfindungen, solche kosmetischen und dermatologischen Zubereitungen zu erstellen, deren hauptsächlicher Zweck nicht der Schutz vor Sonnenlicht ist, die aber dennoch einen Gehalt an UV-Schutzsubstanzen enthalten. So werden z. B. in Tagescremes gewöhnlich UV-A- bzw. UV-B-Filtersubstanzen eingearbeitet.

Auch stellen UV-Schutzsubstanzen, ebenso wie Antioxidantien und, gewünschtenfalls, Konservierungsstoffe, einen wirksamen Schutz der Zubereitungen selbst gegen Verderb dar.

Vorteilhaft können erfindungsgemäße Zubereitungen außerdem als Grundlage für kosmetische Desodorantien bzw. Antitranspirantien eingesetzt werden, so dass die vorliegende Erfindung in einer besonderen Ausführungsform als Grundlage für kosmetische Desodorantien betrifft.

Kosmetische Desodorantien dienen dazu, Körpergeruch zu beseitigen, der entsteht, wenn der an sich geruchlose frische Schweiß durch Mikroorganismen zersetzt wird. Den üblichen kosmetischen Desodorantien liegen unterschiedliche Wirkprinzipien zugrunde.

In sogenannten Antitranspirantien kann durch Adstringentien - vorwiegend Aluminiumsalze wie Aluminiumhydroxychlorid (Aluchlorhydrat) - die Bildung des Schweißes reduziert werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. Der Schweißfluß selbst wird dadurch nicht beeinflußt, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich.

Alle für Desodorantien bzw. Antitranspirantien gängigen Wirkstoffe können vorteilhaft genutzt werden, beispielsweise Geruchsüberdecker wie die gängigen Parfümbestandteile, Geruchsabsorber, beispielsweise die in der Patentoffenlegungsschrift DE-P 40 09 347 beschriebenen Schichtsilikate, von diesen insbesondere Montmorillonit; Kaolinit, llit, Beidellit, Nontronit, Saponit, Hectorit, Bentonit, Smectit, ferner beispielsweise Zinksalze der Ricinolsäure. Keimhemmende Mittel sind ebenfalls geeignet, in die erfindungsgemäßen Emulsionen eingearbeitet zu werden. Vorteilhafte Substanzen sind zum Beispiel 2,4,4'-Trichlor-2'-hdroxydiphenylether (Irgasan), 1,6-Di-(4-chlorphenylbiguanido)-hexan (Chlorhexidin), 3,4,4'-Trichlorcarbanilid, quaternäre Ammoniumverbindungen, Nelkenöl, Minzöl, Thymianöl, Triethylcitrat, Farnesol (3,7,11-Trimethyl-2,6,10-dodecatrien-1-ol) sowie die in den Patentoffenlegungsschriften DE-37 40 186, DE-39 38 140, DE-42 04 321, DE-42 29 707, DE-43 09 372, DE-44 11 664, DE-195 41 967, DE-195 43 695, DE-195 43 696, DE-195 47 160, DE-196 02 108, DE-19602110, DE-19602111, DE-196 31 003, DE-196 31 004 und DE-196 34 019 und den Patentschriften DE-42 29 737, DE-42 37 081, DE-43 24 219, DE-44 29 467, DE-44 23 410 und DE-19516705 beschriebenen Wirkstoffe bzw. Wirkstoffkombinationen. Auch Natriumhydrogencarbonat ist vorteilhaft zu verwenden.

Die Liste der genannten Wirkstoffe bzw. Wirkstoffkombinationen, die in den erfindungsgemäßen Emulsionen oder Gele verwendet werden können, soll selbstverständlich nicht limitierend sein.

Die erfindungsgemäßen kosmetischen Desodorantien können in Form von aus normalen Behältern auftragbaren wasserhaltigen, kosmetischen Zubereitungen vorliegen.

Die Menge der Antitranspiranswirkstoffe oder Desodorantien (eine oder mehrere Verbindungen) in den Zubereitungen beträgt vorzugsweise 0,01 bis 30 Gew.-%, besonders bevorzugt 0,1 - 20 Gew.-%, insbesondere 1 - 10 Gew.-%, bezogen auf das Gesamtgewicht der Zubereitung.

Die erfindungsgemäßen Formulierungen sind ferner hervorragende Vehikel für dermatologische Wirkstoffe. Insbesondere eignen sie sich als Träger für gegen Akne wirksame Substanzen. Akne ist eine Hauterkrankung mit verschiedenen Formen und Ursachen, gekennzeichnet durch nicht entzündliche und entzündliche Knötchen, ausgehend von verstopften Haarfollikeln (Komedonen), die zur Pustel-, Abszeß- und Narbenbildung führen kann. Am häufigsten ist die Acne vulgaris, die vorwiegend in der Pubertät auftritt. Ursächliche Bedingungen für die Acne vulgaris sind die Verhornung und Verstopfung der Haarfollikel-Mündung, die vom Blutspiegel der männlichen Sexualhormone abhängige Talgproduktion und die Produktion freier Fettsäuren und gewebeschädigender Enzyme durch Bakterien (*Propionibacterium acnes*).

Daher ist es vorteilhaft, den erfindungsgemäßen Zubereitungen gegen Akne wirksame Substanzen zuzugeben, die beispielsweise gegen *Propionibacterium acnes* wirksam sind (etwa solche, die in DE-OS 42 29 707, DE-OS 43 05 069, DE-OS 43 07 976, DE-OS 43 37 711, DE-OS 43 29 379 beschrieben werden), aber auch andere gegen Akne wirksame Substanzen, beispielsweise all-trans-Retinsäure, 13-cis-Retinsäure und verwandte Stoffe) oder antientzündliche Wirkstoffe, beispielsweise Batylalkohol (α-Octadecylglycerylether), Selachylalkohol (α-9-Octadecenylglycerylether), Chimylalkohol (α-Hexadecylglycerylether) und/oder Bisabolol sowie Antibiotika und/oder Keratolytika.

Keratolytika sind Stoffe, die verhornte Haut (wie z. B. Warzen, Hühneraugen, Schwielen und dergleichen mehr) erweichen, damit sich diese leichter entfernen läßt oder damit sie abfällt bzw. sich auflöst.

Alle gängigen gegen Akne wirksamen Substanzen können vorteilhaft genutzt werden, insbesondere Benzoylperoxid, Bituminosulfonate (Ammonium-, Natrium- und CalciumSalze von Schieferöl-Sulfonsäuren), Salicylsäure (2-Hydroxybenzoesäure), Miconazol (1-[2-(2,4-Dichlorbenzyloxy)-2-(2,4-dichlorphenyl)-ethyl]-imidazol) und Derivate, Adapalen (6-[3-(1-Adamantyl)-4-methoxyphenyl]-2-naphthoesäure), Azelainsäure (Nonandisäure), Mesulfen (2,7--Dimethylthianthren, C₁₄H₁₂S₂) sowie Aluminiumoxid, Zinkoxid und/oder feinverteilter Schwefel.

Die Menge der Antiaknemittel (eine oder mehrere Verbindungen) in den Zubereitungen beträgt vorzugsweise 0,01 bis 30 Gew.-%, besonders bevorzugt 0,1 - 20 Gew.-%, insbesondere 1 - 10 Gew.-%, bezogen auf das Gesamtgewicht der Zubereitung.

### Beispiele:

Die nachfolgenden Beispiele sollen die vorliegende Erfindung verdeutlichen, ohne sie einzuschränken. Die Zahlenwerte in den Beispielen bedeuten Gewichtsprozente, bezogen auf das Gesamtgewicht der jeweiligen Zubereitungen.

### Allgemeine Vorschrift zur Herstellung:

Für eine Typische O/W-Emulsion könnte eine technische Herstellungsvorschrift im Chargenprozess wie folgt aussehen: Die Fettphase A wird auf 75-80°C erhitzt und im Rührkessel vorgelegt. Separat wird eine schichtsilikathaltige Wasserphase B in einem Rührbehälter auf 75°C erwärmt und homogenisiert.
Anschließend wird Phase B unter Unterdruck und Rühren in die kräftig gerührte Phase A eingetragen. Anschließend wird die gebildete Dispersion unter Vakuum homogenisiert, und langsamen Rühren auf 50 °C abgekühlt. Bei dieser Temperatur erfolgt nochmals ein Homogenisierungsschritt, nach dem auf 35 °C abgekühlt wird. Bei dieser Temperatur werden dann die Wirkstoffphase C und/oder das Parfum D zugegeben und auf 30°C kaltgerührt.

### ÖI-in-Wasser-Emulsion

| | **1** | **2** | **3** | **4** | **5** |
|---|---|---|---|---|---|
| Glycerylsterat | 1,0 | --- | -- | 3,0 | 5,0 |
| PEG-40-Stearat | 10,0 | --- | 5 | -- | -- |
| Stearinsäure-Na-Salz | --- | 5,5 | --- | --- | 2,5 |
| Sorbitanstearat | --- | 1,5 | 3 | --- | --- |
| Cyclomethicon | 1 | 2,5 | 5 | 7,5 | 3 |
| Dimethicon | 5,0 | 3,0 | 5,0 | 2,0 | 15,0 |
| Behenylalkohol | 1 | | 2 1 | 1 | --- |
| Stearylalkohol | | 1 | | 1 | --- |
| Cetylstearylalkohol | | | 1 | 1 | --- |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Schichtsilikat | 0,1 | --- | 0,25 | 1,0 | 0,5 |
| Panthenol | 0,5 | 1,0 | 0,75 | 0,25 | 0,1 |
| Glycerin | 3,0 | 5,0 | --- | --- | --- |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| modifizierte Stärke | --- | 2,5 | --- | 0,15 | -- |
| Wasser | ad 100 | ad 100 | ad 100 | ad 100 | ad 100 |

### ÖI-In-Wasser-Emulsionen

| | **6** | **7** | **8** | **9** | **10** |
|---|---|---|---|---|---|
| Polyethylenglycol(21 ) stearylether | 1 | -- | 2,5 | 2 | 1,5 |
| Polyethylenglycol(2)stearylether | 1 | -- | 0,5 | 3 | 1,5 |
| Cetearylglucosid | --- | 8 | --- | --- | --- |
| Cyclomethicon | 2,5 | 3 | 12,5 | 2 | --- |
| Behenylalkohol | 1 | 0,5 | --- | 1 | --- |
| Stearylalkohol | 1,5 | 0,75 | --- | 2 | --- |
| Cetylstearylalkohol | 0,3 | 1,0 | --- | --- | 2 |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Schichtsilikat | 0,5 | 1,0 | 1,5 | 3,75 | 1,5 |
| Panthenol | 0,5 | 1,0 | 0,75 | 0,25 | 0,1 |
| Glycerin | 3,0 | 5,0 | --- | --- | --- |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| Benzylalkohol | --- | --- | 0,05 | --- | 0,1 |
| modifizierte Stärke | --- | 2,5 | --- | 0,15 | -- |
| Wasser | ad 100 | ad 100 | ad 100 | ad 100 | ad 100 |

### Silikon In Wasser-Emulsion

| | **11** | **12** | **13** | **14** | **15** |
|---|---|---|---|---|---|
| Bis-PEG/PPG-16/16 PEG/PPG16/16 Dimethicone, Caprylic/Caprictriglycerid | 1,0 | 2,0 | 8,0 | 3,0 | 5,0 |
| Cyclomethicon | 12,5 | 15 | 25,0 | 10,0 | 7,5 |
| Dimethicon | 5,0 | 15,0 | 5,0 | 12,0 | 15,0 |
| Schichtsilikat, modifiziert | 0,5 | 0,75 | 1,0 | 2,0 | 0,25 |
| Octyldodecanol | 0,5 | 1,0 | 0,75 | 3,0 | 0,25 |
| Glycerin | 5,0 | 7,5 | 10,0 | 3,0 | 1,0 |
| Panthenol | 0,5 | 1,0 | 0,75 | 0,25 | 0,1 |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| modifizierte Stärke | --- | 2,5 | --- | 0,15 | -- |
| Wasser | ad 100 | ad 100 | ad 100 | ad 100 | ad 100 |

### ÖI-in-Wasser-Emulsion

| | **16** | **17** | **18** | **19** | **20** |
|---|---|---|---|---|---|
| Glycerylsterat Selbstemulgierend | 1,0 | --- | -- | 3,0 | 5,0 |
| PEG-100-Stearat | 10,0 | --- | 5 | -- | -- |
| Glycerylstearatcitrat | --- | 5,5 | --- | --- | 2,5 |
| Sorbitanstearat | --- | 1,5 | 3 | --- | --- |
| Cyclomethicon | 1 | 2,5 | 5 | 7,5 | 3 |
| Dimethicon | 5,0 | 13,0 | 5,0 | 12,0 | 15,0 |
| Behenylalkohol | 1 | | 2 | 1 | --- |
| Stearylalkohol | | 1 | | 1 | --- |
| Cetylstearylalkohol | | | 1 | 1 | --- |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Schichtsilikat | 0,1 | 5,5 | 2,5 | 1,0 | 0,5 |
| Panthenol | 0,5 | 1,0 | 0,75 | 0,25 | 0,1 |
| Glycerin | 3,0 | 5,0 | --- | --- | --- |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| lodopropynylbutylcarbamat | --- | --- | 0,05 | --- | 0,1 |
| modifizierte Stärke | --- | 2,5 | --- | 0,15 | -- |
| Wasser | ad 100 | ad 100 | ad 100 | ad 100 | ad 100 |

### ÖI-In-Wasser-Emulsion

| | **26** | **27** | **28** | **29** | **30** |
|---|---|---|---|---|---|
| Stearinsäure, Na-Salz | 0,75 | 1,5 | 2,75 | 3,5 | 2,5 |
| Sorbitanstearat | --- | 1,5 | 3 | --- | --- |
| Cyclomethicon | 1 | 2,5 | 5 | 7,5 | 3 |
| Behenylalkohol | 1 | --- | 0,2 | 1 | --- |
| Stearylalkohol | --- | 1 | --- | 1 | --- |
| Cetylstearylalkohol | --- | --- | 1 | 1 | --- |
| Mineralöl | 0,5 | 0,75 | 1,0 | 2,0 | 0,25 |
| Octyldodecanol | 0,5 | 1,0 | 0,75 | 3,0 | 0,25 |
| Schichtsilikat | 1,0 | 2,5 | 5,0 | 1,0 | 0,5 |
| Harnstoff | 0,5 | 1,0 | 0,75 | 0,25 | 0,1 |
| Glycerin | 3,0 | 5,0 | --- | --- | --- |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| Wasser | ad 100 | ad 100 | ad 100 | ad 100 | ad 100 |

### Hydrodispersionsgele

| | **31** | **32** | **33** | **34** | **35** |
|---|---|---|---|---|---|
| Schichtsilikat | 1,0 | 1,5 | 2,5 | 4,0 | 2,5 |
| PEG-40 -Stearat | 0,5 | --- | 0,75 | -- | -- |
| Cyclomethicon | 1 | 2,5 | 5 | 7,5 | 3 |
| Dimethicon | 0,5 | 1,0 | 0,75 | 1,25 | 1,0 |
| Stearylalkohol | --- | 1 | --- | 1 | --- |
| Cetylstearylalkohol | --- | --- | 1 | 1 | --- |
| hydriertes Polyisobuten | 0,5 | 0,75 | 1,0 | 2,0 | 0,25 |
| Octyldodecanol | 0,5 | 1,0 | 0,75 | 3,0 | 0,25 |
| Panthenol | 0,5 | 1,0 | 0,75 | 0,25 | 0,1 |
| Glycerin | 3,0 | 5,0 | --- | --- | --- |
| Parfum | q,s, | q,s, | q,s, | q,s, | q,s, |
| Methylparaben | 0,4 | 0,1 | 0,05 | 0,3 | 0,4 |
| Propylparaben | 0,3 | 0,4 | 0,25 | 0,15 | --- |
| Ethanol | --- | --- | 5,0 | --- | 3,5 |
| Wasser | ad 100 | ad 100 | ad 100 | ad 100 | ad 100 |

## Patentansprüche

1. Gelförmige kosmetische oder dermatologische Zubereitung, die ein feindisperses System vom Typ Emulsion oder Hydrodispersion darstellt, **dadurch gekennzeichnet, dass** sie
- eine Wasserphase,
- mindestens ein Schichtsilikat, insbesondere ein aufbereitetes natürliches oder synthetisches Schichtsilikat,
- gegebenenfalls ein modifiziertes Schichtsilikat, welches sowohl hydrophile als auch lipophile Eigenschaften zeigt
- einen oder mehrere Emulgatoren aus der Gruppe anionische oder nichtionische Emulgatoren, mit eine Gesamtkonzentration von 1,0 bis 5,5 Gew.-%, bezogen auf die gesamte Zubereitung,
- eine Ölphase und/oder Silikonölphase, mit eine Gesamtkonzentration von 0,001 bis 20 Gew.-%, bezogen auf die gesamte Zubereitung, enthält und
- im ruhenden Zustand eine gelförmige Konsistenz aufweist und unter Scherbeanspruchung einer reversiblen Viskositätsänderung, insbesondere einer Viskositätserniedrigung, unterliegt.

2. Zubereitungen nach Anspruch 1, **dadurch gekennzeichnet, dass** die Zubereitungen sprühbar und pumpbar sind.

3. Zubereitungen nach einem der vorhergehenden Ansprüche, **dadurch gekennzeichnet, dass** weitere kosmetische oder pharmazeutische Hilfs-, Zusatz- und/oder Wirkstoffe enthalten sind.

4. Zubereitungen nach einem der vorhergehenden Ansprüche, **dadurch gekennzeichnet, dass** mindestens ein Schichtsilikat ein Natriummagnesiumsilikat oder ein Magnesiumaluminiumsilikat, insbesondere ein synthetisches Natriummagnesiumsilikat oder Magnesiumaluminiumsilikat ist.

5. Zubereitungen nach einem der vorhergehenden Ansprüche, **dadurch gekennzeichnet, dass** mindestens ein Schichtsilikat ein Schichtsilikat aus der Gruppe Laponite®, Veegum® oder OPTIGEL® ist.

6. Zubereitungen nach einem der vorhergehenden Ansprüche, **dadurch gekennzeichnet, dass** der Gehalt an einem oder mehreren aufbereiteten natürlichen Schichtsilikat und/oder synthetischen Schichtsilikat zwischen 0,05 Gew.-% und 10 Gew.-% ist, vorteilhaft zwischen 0,5 und 4 Gew.-%, bezogen auf das Gesamtgewicht der Zubereitungen.

7. Zubereitungen nach einem der vorhergehenden Ansprüche, **dadurch gekennzeichnet, dass** neben einem oder mehreren aufbereiteten natürlichen bzw. synthetischen Schichtsilikaten und gegebenenfalls modifizierten Schichtsilikaten weitere Pigmente enthalten sind, insbesondere modifizierte Polysaccharide und/oder mikrofeine Polymerpartikel und/oder mikronisierte, anorganische Pigmente, die gewählt werden aus der Gruppe der amphiphilen Metalloxide, insbesondere aus der Gruppe Titandioxid, Zinkoxid, Eisenoxide bzw. Eisenmischoxide, Siliciumdioxid bzw. Silicate, wobei die Pigmente sowohl einzeln als auch im Gemisch vorliegen können.

8. Zubereitungen nach einem der vorhergehenden Ansprüche, enthaltend einen oder mehrere Zusatz- oder Wirkstoffe, gewählt aus der Gruppe der Antioxidantien und/oder der Gruppe der UV-Filtersubstanzen.

9. Zubereitungen nach einem der vorhergehenden Ansprüche, enthaltend einen oder mehrere Zusatz- oder Wirkstoffe, gewählt aus der Gruppe der Adstringentien und/oder antimikrobiell wirksamen Stoffe und/oder gegen Akne wirksamen Stoffe.

10. Kosmetisches Produkt, **dadurch gekennzeichnet, dass** es
- einen Zerstäubereinrichtung, insbesondere eine Zerstäuberpumpe,
- einen mit der Zerstäubereinrichtung kombinierbaren Produktbehälter und
- eine Zubereitung nach mindesten einem der vorgehenden Ansprüche enthält,
wobei sich die Zubereitung im Produktbehälter befindet und durch Betätigung der Zerstäubereinrichtung entnommen werden kann.

11. Verwendung einer Zubereitung nach mindestens einem der Ansprüche 1 bis 9 als sprühbares Gel, insbesondere als sprühbares gelförmiges Kosmetikum.

12. Verfahren zur Herstellung geförmiger kosmetischer oder dermatologischer Zubereitungen nach Anspruch 1 bis 9, bei dem in einem ersten Schritt ein Schichtsilikat in Wasser unter Einwirkung hoher Scherkräfte dispergiert wird, so dass eine homogene Phase entsteht, die in einem zweiten Schritt unter Rühren in eine Phase eingetragen wird, die die weiteren Bestandteile der Zubereitung enthält, so dass eine homogene Verteilung innerhalb der alle Komponenten umfassenden Zubereitung vorliegt.
